# EUROPÄISCHE PATENTANMELDUNG

(11) **EP 1 949 935 A1**
(43) Veröffentlichungstag der Anmeldung: **30.07.2008**
(21) Anmeldenummer: 08100862.5
(22) Anmeldetag: 24.01.2008
(51) Int. Cl.: A61N 5/10

(54) **Partikeltherapie-Anlage**

(30) Priorität: 25.01.2007 DE 102007003878
(71) Anmelder: SIEMENS AKTIENGESELLSCHAFT, 80333 München (DE)
(72) Erfinder: Kaiser, Werner, 91052 Erlangen (DE); Use, Tim, 90469 Nürnberg (DE)

(57) **Zusammenfassung**

Eine Partikeltherapie-Anlage (2) umfasst eine Bestrahlungseinheit (16) aufweisende und um eine Drehachse (D) drehbare Gantry (4), die einen Bestrahlungsraum (6) mit einem beweglichen Bodensegment(22) umschließt, in welchem Bestrahlungsraum (6) ein Patiententisch (8) positionierbar ist. Um eine Bestrahlung eines Patienten (12) aus allen Winkellagen zu ermöglichen, ist das Bodensegment (22) auf der Gantry (4) derart gelagert, dass bei einer Drehung der Gantry (4) das Bodensegment (22) in einer waagerechten Nullposition bleibt und bei Bedarf eine Bewegung um die Drehachse (D) der Gantry (4) ausführt. Für viele Winkellagen bleibt also das Bodensegment 22 in der Nullposition, wodurch während der Behandlung des Patienten (12) ein tragfähiger Boden im Bestrahlungsraum (6) vorhangen ist. Für eine Bestrahlung des Patienten (12) von unterhalb des Patiententisches (8) wird das Bodensegment (22) um die Drehachse (D) verschoben, um Platz für die Bestrahlungseinheit (16) frei zu machen.

## Beschreibung

Die Erfindung betrifft eine Partikeltherapie-Anlage umfassend eine Bestrahlungseinheit aufweisende und um eine Drehachse drehbare Gantry, die einen Bestrahlungsraum mit einem beweglichen Bodensegment umschließt, in welchem Bestrahlungsraum ein Patiententisch positionierbar ist.

Bei einer Partikeltherapie, insbesondere von Krebserkrankungen, wird in einem geeigneten Beschleuniger ein Partikelstrahl beispielsweise aus Protonen oder Schwerionen erzeugt. Der Partikelstrahl wird in einem Strahlungskanal geführt und tritt über ein Austrittsfenster des Strahlungskanals in einen Bestrahlungsraum ein. In vielen Fällen ist wegen der aufwändigen Strahlungsführung lediglich ein ortsfestes Strahlaustrittsfenster vorgesehen. Bei einigen Anlagen ist jedoch eine drehbare Gantry mit einem Austrittsfenster vorgesehen. Wegen der aufwändigen Strahlungsführung ist die Gantry sehr großvolumig aufgebaut. Die Gantry umschließt den etwa zylinderförmigen Bestrahlungsraum, in den ein Patiententisch hineingefahren wird. Für eine möglichst präzise Behandlung muss das zu bestrahlende Gewebe des Patienten möglichst genau im Isozentrum (der Treffpunkt des Strahles bei der Rotation der Gantry) der Anlage positioniert werden.

Am Ende des Strahlungskanals sind in einer auch als Nozzle bezeichnet Bestrahlungseinheit unmittelbar vor dem Austrittsfenster üblicherweise mindestens ein Strahlendetektor sowie passive Strahlelemente angeordnet. Um den Patienten auch von unten bestrahlen zu können, ist die Gantry idealerweise um 360° um den Patienten rotierbar. Hierbei besteht das Problem, dass die Bestrahlungseinheit auch in dem Bereich unterhalb des Patienten rotierbar sein muss. Für diesen Zweck öffnet sich der Boden des Bestrahlungsraums und passt sich an die Rotation der Bestrahlungseinheit an.

In der WO 2004/026401 A1 ist eine Bestrahlungskammer beschrieben, die ein halboffener Raum in Zimmergröße ist. Der Boden dieses Raums ist fest installiert mit Ausnahme eines ca. 50 cm breiten Schlitzes für die Führung einer Bestrahlungseinheit. Der Schlitz ist mit einer beidseitig geführten Rollabdeckung zugedeckt. Die Gantry ist nur um 180° drehbar.

Aus der EP 1 402 923 A1 ist ein weiteres Partikelbestrahlungsgerät zu entnehmen, bei dem die Wand und der Boden eines Bestrahlungsraums aus gelenkig miteinander verbundenen Platen bestehen und bei der Rotation der Gantry um den Patiententisch geschoben werden. Bei dieser Ausführung ist die Tragfähigkeit des Bodens stark eingeschränkt.

Der Erfindung liegt die Aufgabe zugrunde eine Partikeltherapie-Anlage mit einem einfachen Aufbau anzugeben, bei dem der Patient aus allen Winkellagen bestrahlt werden kann.

Die Aufgabe wird erfindungsgemäß gelöst durch eine Partikeltherapie-Anlage, umfassend eine Bestrahlungseinheit aufweisende und um eine Drehachse drehbare Gantry, die einen Bestrahlungsraum mit einem beweglichen Bodensegment umschließt, in welchem Bestrahlungsraum ein Patiententisch positionierbar ist, wobei das Bodensegment derart mit der Gantry gekoppelt ist, dass bei einer Drehung der Gantry das Bodensegment in einer waagerechten Nullposition bleibt und bei Bedarf eine Bewegung um die Drehachse der Gantry ausführt.

Die Erfindung basiert auf der Überlegung, dass eine freie Bewegung der Bestrahlungseinheit zum Bestrahlen eines Patienten aus allen Winkellagen möglich ist, indem das Bodensegment und die Gantry zueinander relativ beweglich gelagert sind. Das Bodensegment ist also derart mit der Gantry gekoppelt, dass eine Rotation der Gantry nicht zwangläufig eine Bewegung des Bodensegments auslöst. Unter Bodensegment wird hierbei insbesondere ein im zusammengebauten Zustand einstückiges Element mit einem einfachen Aufbau und einer geeignet gewählten mechanischen Belastbarkeit verstanden, welches sowohl massiv als auch nach Art eines hohlen Gerüstes ausgebildet werden kann. Seine begehbare Seite ist fest und erstreckt sich insbesondere vollflächig zwischen den Wänden des Bestrahlungsraums. Wenn keine Kollisionsgefahr zwischen dem Bodensegment und der Bestrahlungseinheit besteht, bleibt das Bodensegment in der waagerechten Nullposition und gewährt somit den Zugang zum Patienten. Und bei einer möglichen Kollision mit der Bestrahlungseinheit wird das Bodensegment um die Drehachse der Gantry verschoben, so dass Platz für die Bestrahlungseinheit unterhalb des Patiententisches frei gemacht wird. Somit steht für sehr viele Bestrahlungswinkel (z.B. bis +90°/-90° ausgehend von einer vertikalen Lage der Bestrahlungseinheit über dem Bodensegment) im Bestrahlungsraum ein stationärer, spaltfreier Boden mit ausreichender Tragfähigkeit zur Verfügung, über den der Zugang zum Patienten für Personal und Geräte gewährleistet ist. Erst bei Bestrahlungswinkeln größer als +/-90° muss das Bodensegment weggefahren werden.

Bei der vorgeschlagenen Konstruktion der Partikeltherapie-Anlage ist zum Platzieren des Patiententisches innerhalb des Bestrahlungsraums insbesondere ein auf einem außerhalb des Bestrahlungsraums befindlichen Festbodens befestigter Roboterarm vorgesehen. Mit Hilfe des Roboterarms kann der Patiententisch in den von der Gantry umschlossenen Bestrahlungsraum hineingefahren und gehalten werden, ohne dass der Patiententisch Kontakt mit dem Bodensegment hat. Somit kann das Bodensegment zum Vermeiden einer Kollision mit der Bestrahlungseinheit aus der Nullposition verfahren werden, ohne dass dies eine Auswirkung auf die Position des Patiententisches hat.

Bevorzugt ist das Bodensegment auf der Gantry gelagert, d.h. das Bodensegment stützt sich ausschließlich auf die Gantry. Zur beweglichen Lagerung des Bodensegments auf der Gantry sind insbesondere Rollenlager vorgesehen, die sich auf eine Seitenwand der Gantry abstützen. Alternativ kann die Führung des Bodensegments auch z.B. über eine Schiene auf der Seitenwand der Gantry erfolgen. Die Seitenwand ist nach Art einer Zylindermantelfläche aus einem tragfähigen Material ausgebildet und begrenzt den Bestrahlungsraum. Bei einer Rotation der Gantry zum Verfahren der aus ihr herausragenden Bestrahlungseinheit um die Drehachse, d.h. bei einer Rotation der Seitenwand, wälzen sich die Rollenlager auf der bewegenden Seitenwand, so dass das Bodensegment ohne die Einwirkung einer äußeren Kraft weiterhin in der waagerechten Nullposition bleibt. Ein Verschieben des Bodensegments erfolgt unter der Einwirkung einer äußeren Kraft, wobei er mittels der Rollenlager auf der Seitenwand zur Seite gerollt wird oder seine Position bezüglich der Seitenwand bleibt fest und er wird mit der Gantry mitgedreht. Die Gantry ist hierbei insbesondere derart ausgebildet, dass die Bestrahlungseinheit ausgehend von einer vertikalen Positionierung über dem in der Nullposition befindlichen Bodensegment, um einen Winkelbereich von +/- 180° im und entgegen dem Uhrzeigersinn um die Drehachse rotierbar ist, wodurch ein Drehwinkelbereich von 360° abgedeckt ist.

Um die Lagerung und die Bewegung des Bodensegments auf der Gantry zu begünstigen, weist das Bodensegment bevorzugt im Querschnitt gesehen eine maximal halbkreisförmige, an die Gantry angepasste Geometrie auf. Hierbei kann das Bodensegment einen massiven kreissegmentförmigen Körper oder alternativ einen im Querschnitt gesehen hohlen kreissegmentförmigen Rahmen darstellen, der sich insbesondere bis unterhalb eines Isozentrums der Gantry erstreckt, um ein einwandfreies Positionieren des Patienten im Isozentrum zu ermöglichen.

Gemäß einer bevorzugten Ausgestaltung ist ein Reibelement zum Festhalten des Bodensegments in der waagerechten Nullposition vorgesehen. Ein Reibschluss, d.h. das Festhalten des Bodensegments mit Hilfe einer Reibkraft, stellt eine einfach zu realisierende Möglichkeit zum Halten des Bodenelements in der waagerechten Nullposition dar. Hierbei ist die Reibkraft des Reibelements derart ausgelegt, dass sie größer ist als die Reibkräfte zwischen den Rollenlagern und der Seitenwand der Gantry, so dass beim Drehen der Gantry ohne die Einwirkung einer äußeren Kraft ein Mitdrehen des Bodensegments verhindert wird.

Gemäß einer weiteren bevorzugten Ausgestaltung ist das Reibelement elastisch gelagert. Hierbei wird der Vorteil erzielt, dass ein kleiner Toleranzbereich bei der Bewegung des Bodensegments geschaffen wird, indem die Reibkräfte des Reibelements auf das Bodensegment wirken und es zurück in die Nullposition bringen. Die elastische Lagerung kann beispielsweise über eine Feder oder einen massiven Körper aus einem biegsamen Material wie z.B. Gummi erfolgen.

Bevorzugt ist ein mit dem Bodensegment verbundener Ausleger vorgesehen, an dem das Reibelement angreift. Durch den Ausleger wird insbesondere eine große Fläche zum Kontakt zwischen dem Reibelement und dem Bodensegment geboten, durch die der Toleranz- bzw. Wirkbereich des Reibelements vergrößert wird.

Vorteilhafterweise ist zur Führung des Auslegers eine um den Bestrahlungsraum umlaufende Kulisse vorgesehen. Dank der Kulisse ist der Ausleger außerhalb des Bestrahlungsraums angeordnet, so dass der Ausleger insbesondere keinen direkten Kontakt zu den beweglichen Teilen der Gantry hat. Somit ist zum einen die Positionierung des Patiententisches durch den Ausleger nicht behindert und zum anderen kann der Ausleger unabhängig von der Drehung der Gantry festgehalten werden.

Nach einer bevorzugten Variante ist die Gantry derart ausgebildet, dass die Bestrahlungseinheit bei ihrer Drehung oder bei einer definierten Drehposition das Bodensegment in Drehrichtung schiebt. Diese Variante zeichnet sich dadurch aus, dass zum Verfahren des Bodensegments kein zusätzlicher Antrieb bzw. keine eigene Steuerung erforderlich ist. Das Bodensegment bleibt also solange in der Nullposition, bis es bei der Drehung der Bestrahlungseinheit von dieser mitgenommen und in Drehrichtung verschoben wird. Hierbei ist das Bodensegment beispielsweise nach Art einer Leichtmetallkonstruktion ausgebildet, so dass es keine große Zusatzlast für den Antrieb der Gantry im Vergleich zu der schweren Bestrahlungseinheit darstellt.

Um einen Stoß zwischen der Bestrahlungseinheit und dem Bodensegment bei der Drehung der Bestrahlungseinheit zu mildern und somit eine günstige graduelle Krafteinleitung in das Bodensegment zu gewähren, ist zweckdienlicherweise im Bereich der Kontaktstelle zwischen der Bestrahlungseinheit und dem Bodenelement an der Bestrahlungseinheit und/oder am Bodensegment ein Pufferelement angeordnet.

Vorteilhafterweise ist die Kontaktstelle zwischen der Bestrahlungseinheit und dem Bodensegment außerhalb des Bestrahlungsraums angeordnet. Dadurch wird der Bestrahlungsraum nicht unnötig durch die für die Ausbildung der Kontaktstelle erforderliche Komponenten, wie z.B. Pufferelemente, verkleinert.

Weiterhin von Vorteil ist, dass beidseitig des Bodenelements zwei geneigte Flügel vorgesehen sind, auf denen das Pufferelement angeordnet ist. Durch die geneigte Stellung jeder der Flügel ist eine Anordnung des Pufferelements ermöglicht, bei der ein großflächiger Kontakt zwischen dem Bodensegment und der Bestrahlungseinheit erfolgt, der die Krafteinleitung in das Bodensegment begünstigt.

In einer bevorzugten Ausführungsform ist eine Rückwand des Bestrahlungsraums dafür ausgebildet, mit der Gantry mitzurotieren. Dies ermöglicht eine kostengünstige und einfach zu realisierende Ausführung der Rückwand als eine Verkleidung ohne mechanische Belastbarkeit.

Alternativ zur Ausgestaltung, bei der das Bodensegment von der rotierenden Bestrahlungseinheit in Drehrichtung vorgeschoben wird oder in Kombination mit dieser Ausgestaltung ist bevorzugt eine Steuereinheit vorgesehen, die dafür ausgebildet ist, das Bodensegment unabhängig von der Position der Bestrahlungseinheit zu verfahren. Hierbei kann eine Bewegung des Bodensegments erfolgen, die völlig entkoppelt von der Drehbewegung der Bestrahlungseinheit ist, so dass ein Verfahren des Bodensegments um die Drehachse auch ohne einen direkten Kontakt zwischen dem Bodensegment und der Bestrahlungseinheit erfolgt. Diese Ausführung ist besonders schonend für den Antrieb der Gantry, der keine Zusatzlast aufnehmen muss.

Ein Ausführungsbeispiel der Erfindung wird anhand einer Zeichnung näher erläutert. Hierin zeigen:
- FIG 1: eine Vorderansicht auf eine Partikeltherapie-Anlage mit einem zylinderförmigen Bestrahlungsraum, und
- FIG 2: einen Längsschnitt durch die Partikeltherapie-Anlage gemäß FIG 1.

Gleiche Bezugszeichen haben in den Figuren die gleiche Bedeutung.

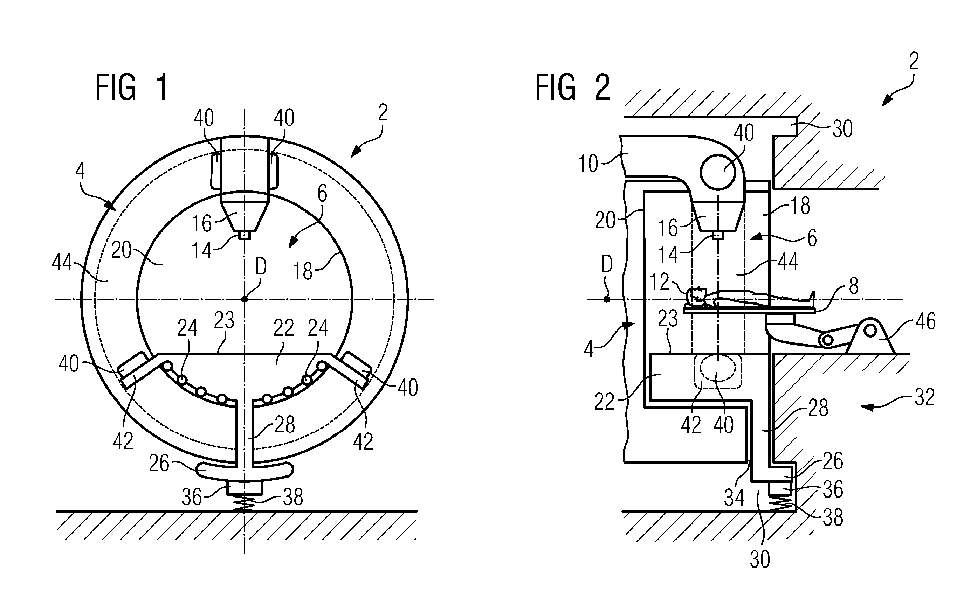

In FIG 1 und FIG 2 ist aus verschiedenen Perspektiven eine Partikeltherapie-Anlage 2 mit einer um eine Drehachse D drehbaren Gantry 4 dargestellt. Die Drehachse D verläuft bei der Darstellung der Partikeltherapie-Anlage gemäß FIG 1 senkrecht zur Zeichnungsebene und ist deshalb durch einen Punkt D angedeutet. Die Gantry 4 umschließt einen etwa zylinderförmigen Bestrahlungsraum 6, in dem ein Patiententisch 8 positionierbar ist, wie aus FIG 2 zu entnehmen ist. Die Gantry 4 umfasst außerdem einen teilweise gezeigten Strahlungskanal 10. Im Strahlungskanal 10 wird ein hier nicht dargestellten Partikelstrahl, beispielsweise ein Schwerionen- oder Protonenstrahl zur Behandlung eines auf dem Patiententisch 8 liegenden Patienten 12 geführt. Der Partikelstrahl tritt über ein Austrittsfenster 14 einer Bestrahlungseinheit 16 in den Bestrahlungsraum 6 ein, wobei die Bestrahlungseinheit 16 aus einer rotierbaren, tragfähigen Seitenwand 18 des Bestrahlungsraums 6 herausragt. Nach hinten ist der Bestrahlungsraum 6 durch eine Rückwand 20 begrenzt, die in diesem Ausführungsbeispiel eine einfache Verkleidung ohne mechanische Belastbarkeit ist und mit der Gantry 4 um die Drehachse D rotiert.

Der Bestrahlungsraum 6 weist weiterhin einen Boden auf, der aus einem einzigen massiven kreisbogenförmigen Bodensegment 22 besteht, das auf der Seitenwand 18 der Gantry 4 gelagert ist. Das Bodensegment 22 hat im gezeigten Ausführungsbeispiel eine waagerechte Nullposition angenommen und bildet somit eine begehbare Bodenfläche 23. Die Tragfähigkeit des Bodensegments 22 ist den Erfordernissen entsprechend gewählt, so dass das Bodensegment 22 einen spaltfreien Bestrahlungsraumboden bildet, der mit einem Gewicht von z.B. etwa 200 kg belastet werden kann.

Das Bodensegment 22 ist über eine Anzahl von Lagern 24, die in diesem Ausführungsbeispiel Rollenlager sind, derart auf der Seitenwand 18 gelagert, dass das Bodensegment 22 und die Seitenwand 18 zueinander relativ beweglich sind. Dies bedeutet, dass die Gantry 4 rotieren kann, wobei das Bodensegment 22 in seiner Nullposition bleibt. Alternativ kann das Bodensegment 22 angetrieben von einer äußeren Kraft über seine Rollenlager 24 bei feststehender Gantry 4 eine Bewegung um die Drehachse D ausführen. Möglich ist auch, dass zum Verfahren des Bodensegments 22 um die Drehachse D seine Lage gegenüber der Gantry 4 fest bleibt und es bei der Rotation der Gantry mitgeführt wird.

Das Bodensegment 22 weist einen Ausleger 26 auf, der über einen sich senkrecht zu der horizontalen Bodenfläche 23 erstreckenden Steg 28 mit dem Bodensegment 22 verbunden ist. Zur Führung des Auslegers 26 und des Steges 28 beim Verfahren des Bodensegments 22 um die Drehachse D sind eine um den Bestrahlungsraum 6 umlaufende Kulisse 30, die insbesondere in einem den Bestrahlungsraum 6 angrenzenden Festboden 32 ausgebildet ist bzw. ein zwischen der Gantry 4 und dem Festboden 32 gebildeter Schacht 34 vorgesehen. Darüber hinaus ist zum Festhalten des Bodensegments 22 in der Nullposition ein Reibelement 36 vorgesehen, das an dem Ausleger 26 angreift und über eine Feder 38 elastisch gelagert ist.

Die in FIG 1 und FIG 2 gezeigte Anordnung hat den Vorteil, dass bei sehr vielen Bestrahlungswinkeln ein ortsfester Boden im Bestrahlungsraum 6 vorhanden ist. Z.B. ist ausgehend von der gezeigten senkrechten Lage der Bestrahlungseinheit 16 oberhalb des Bodensegments 22 eine Rotation der Bestrahlungseinheit 16 um 90° im Uhrzeigersinn sowie entgegen dem Uhrzeigersinn möglich, ohne dass dabei eine Kollision mit dem Bodensegment 22 droht. In diesem Winkelbereich muss also das Bodensegment 22 nicht weggefahren werden. Erst bei einer Auslenkung der Bestrahlungseinheit 16 aus der gezeigten Position um einen Drehwinkel, der größer ist als +/- 90°, erfolgt ein Verschieben des Bodensegments 22 um die Drehachse D. Hierfür kann das Bodensegment 22 einen eigenen Antrieb aufweisen, so dass angesteuert von einer Steuereinheit das Bodensegment 22 um die Drehachse D verfahren wird, noch bevor ein Kontakt zwischen dem Bodensegment 22 und der Bestrahlungseinheit 16 entsteht. In dem gezeigten Ausführungsbeispiel ist allerdings kein separater Antrieb bzw. keine separate Ansteuerung des Bodensegments 22 vorgesehen, sondern es erfolgt eine Zwangsführung des Bodensegments 22 über die Bestrahlungseinheit 16, die bei ihrer Drehung das Bodensegment 22 vor sich schiebt.

Um einen harten Stoß zwischen der Bestrahlungseinheit 16 und dem Bodensegment 22 zu vermeiden, sind sowohl an der Bestrahlungseinheit 16 als auch am Bodensegment 22 im Bereich einer Kontaktstelle zwischen den beiden Pufferelemente 40 angeordnet. Damit ein möglichst großflächiger Kontakt zwischen dem Bodensegment 22 und der Bestrahlungseinheit 16 erfolgt, weist das Bodensegment 22 beidseitig zwei geneigte Flügel 42 auf, die sich außerhalb des Bestrahlungsraums 6 ausstrecken und die Pufferelemente 40 des Bodensegments 22 tragen. Durch die geneigte Stellung der Flügel 42 wird insbesondere ein günstiger Winkel eingestellt, so dass die Pufferelemente 40 des Bodensegments 22 und der Bestrahlungseinheit 16 großflächig aufeinander aufliegen, wenn die Bestrahlungseinheit 16 bei ihrer Drehung das Bodensegment 22 erreicht. Um die Flügel 42 außerhalb des Bestrahlungsraums anordnen zu können, ist in der Seitenwand 18 eine umlaufende Vertiefung 44 vorgesehen, die in den Figuren durch eine gestrichelte Linie angedeutet ist.

Das Bodensegment 22 ist in diesem Ausführungsbeispiel nach Art einer Leichtmetallkonstruktion ausgebildet, so dass der Antrieb der Gantry 4 beim Verschieben des Bodensegments 22 durch die Bestrahlungseinheit 16 möglichst wenig ausgelastet wird. Bei einer Auslenkung der Bestrahlungseinheit 16 ausgehend von ihrer in den Figuren gezeigten Vertikallage um einen Auslenkwinkel, der größer als 180° ist, summieren sich die Komponenten der Gewichtskraft sowohl des Bodensegments 22 als auch der Bestrahlungseinheit 16 die in Richtung zurück zur Nullposition wirken, was eine sehr hohe Belastung für den Antrieb der Gantry 4 bedeutet. Damit der Antrieb nicht unnötig belastet wird, ist bei der Partikeltherapie-Anlage 2 vorgesehen, dass die Bestrahlungseinheit 16 ausgehend von ihrer Vertikallage nur bis 180° im Uhrzeigersinn oder entgegen dem Uhrzeigersinn verfahren wird, wodurch allerdings der gesamte Winkelbereich von 360° abgedeckt ist.

Der Patiententisch 8 wird in diesem Ausführungsbeispiel über einen Roboterarm 46 in den Bestrahlungsraum 6 hineingefahren. Dabei hat der Patiententisch 8 keinen Kontakt mit dem Bodensegment 22. Der Roboterarm 46 ist hierbei ein mehrachsiger Industrieroboterarm mit einer mehrteiligen Mechanik und ist auf dem Festboden 32 montiert ist. Mit Hilfe des Roboterarms 46 wird der Patiententisch 8 translatorisch in die horizontale sowie in die vertikale Richtung bewegt. Hierbei ist ein Drehen des Roboterarms 46 um mehrere Achsen möglich, so dass die Bewegung des Patiententisches 8 sich durch drei translatorische Freiheitsgrade sowie drei Rotationsfreiheitsgrade auszeichnet. Durch die Translations- und Rotationsbewegung des Patiententisches 8 werden die Lage und die Entfernung des Patienten 12 zur Bestrahlungseinheit 16 eingestellt. Während seiner Positionierung in dem Bestrahlungsraum 6 bleibt der Patiententisch 8 in einer horizontalen Lage, damit der Patient 12 stabil liegt.

Die hier beschriebene Partikeltherapie-Anlage 2 zeichnet sich dadurch aus, dass eine Bestrahlung des Patienten 12 von allen Winkellagen möglich ist, wobei ein problemloses Verfahren der Bestrahlungseinheit 16 unterhalb des Patiententisches 8 gewährt ist, indem das Bodensegment 22 über seine bewegliche Lagerung von der Bestrahlungseinheit 16 selbst weggeschoben wird. Diese Ausgestaltung hat den wesentlichen Vorteil, dass kein zusätzlicher Antrieb sowie keine separate Ansteuerung des Bodensegments 22 erforderlich sind. Zudem kann das Bodensegment 22 für sehr viele Bestrahlungswinkel in seiner Nullposition bleiben, wodurch ein begehbarer und befahrbarer Bestrahlungsraumboden auch während der Behandlung des Patienten 12 vorhanden ist.

## Patentansprüche

1. Partikeltherapie-Anlage (2) umfassend eine Bestrahlungseinheit (16) aufweisende und um eine Drehachse (D) drehbare Gantry (4), die einen Bestrahlungsraum (6) mit einem beweglichen Bodensegment (22) umschließt, in welchem Bestrahlungsraum (6) ein Patiententisch (8) positionierbar ist,
**dadurch gekennzeichnet, dass** das Bodensegment (22) mit der Gantry (4) derart gekoppelt ist, dass bei einer Drehung der Gantry (4) das Bodensegment (22) in einer waagerechten Nullposition bleibt und bei Bedarf eine Bewegung um die Drehachse (D) der Gantry (4) ausführt.

2. Partikeltherapie-Anlage (2) nach Anspruch 1,
**dadurch gekennzeichnet, dass** das Bodensegment (22) auf der Gantry (4) gelagert ist.

3. Partikeltherapie-Anlage (2) nach Anspruch 1 oder 2,
**dadurch gekennzeichnet, dass** das Bodensegment (22) im Querschnitt gesehen eine maximal halbkreisförmige, an die Gantry (4) angepasste Geometrie aufweist.

4. Partikeltherapie-Anlage (2) nach einem der vorhergehenden Ansprüche,
**dadurch gekennzeichnet, dass** das Bodensegment (22) mittels eines Reibelements (36) in der waagerechten Nullposition festgehalten wird.

5. Partikeltherapie-Anlage (2) nach Anspruch 4
**dadurch gekennzeichnet, dass** das Reibelement (36) elastisch gelagert ist.

6. Partikeltherapie-Anlage (2) nach Anspruch 4 oder 5,
**dadurch gekennzeichnet, dass** ein mit dem Bodensegment (22) verbundener Ausleger (26) vorgesehen ist, an dem das Reibelement (36) angreift.

7. Partikeltherapie-Anlage (2) nach Anspruch 6,
**dadurch gekennzeichnet, dass** zur Führung des Auslegers (26) eine um den Bestrahlungsraum (6) umlaufende Kulisse (30) vorgesehen ist.

8. Partikeltherapie-Anlage (2) nach einem der vorhergehenden Ansprüche,
**dadurch gekennzeichnet, dass** die Gantry (4) derart ausgebildet ist, dass die Bestrahlungseinheit (16) bei ihrer Drehung ab einer definierten Drehposition das Bodensegment (22) in Drehrichtung schiebt.

9. Partikeltherapie-Anlage (2) nach Anspruch 8,
**dadurch gekennzeichnet, dass** im Bereich einer Kontaktstelle zwischen der Bestrahlungseinheit (16) und dem Bodensegment (22) an der Bestrahlungseinheit (16) und/oder am Bodensegment (22) ein Pufferelement (40) angeordnet ist.

10. Partikeltherapie-Anlage (2) nach Anspruch 9,
**dadurch gekennzeichnet, dass** die Kontaktstelle zwischen der Bestrahlungseinheit (16) und dem Bodensegment (22) außerhalb des Bestrahlungsraums (6) angeordnet ist.

11. Partikeltherapie-Anlage (2) nach Anspruch 9 oder 10,
**dadurch gekennzeichnet, dass** beidseitig des Bodensegments (22) zwei geneigte Flügel (42) vorgesehen sind, auf denen das Pufferelement (40) angeordnet ist.

12. Partikeltherapie-Anlage (2) nach einem der vorhergehenden Ansprüche,
**dadurch gekennzeichnet, dass** eine Rückwand (20) des Bestrahlungsraums (6) dafür ausgebildet ist, mit der Gantry (4) mitzurotieren.

13. Partikeltherapie-Anlage (2) nach einem der vorhergehenden Ansprüche,
**dadurch gekennzeichnet, dass** eine Steuerung vorgesehen sind, die dafür ausgebildet ist, unabhängig von der Position der Bestrahlungseinheit (16) das Bodensegment (22) zu verfahren.
